# EUROPÄISCHE PATENTSCHRIFT

(11) **EP 0 929 790 B1**
(45) Veröffentlichungstag und Bekanntmachung des Hinweises auf die Patenterteilung: **27.02.2002**
(21) Anmeldenummer: 96945472.7
(22) Anmeldetag: 30.09.1996
(51) Int. Cl.: G01B 7/06, G01B 101/00

(54) **SENSOR ZUR BERÜHRUNGSLOSEN DICKENMESSUNG AN FOLIEN**
NON-CONTACT FILM THICKNESS GAUGING SENSOR
CAPTEUR POUR MESURER SANS CONTACT L'EPAISSEUR DE FEUILLES

(43) Veröffentlichungstag der Anmeldung: 21.07.1999
(73) Patentinhaber: Micro-Epsilon Messtechnik GmbH & Co. KG, 94496 Ortenburg (DE)
(72) Erfinder: DUMBERGER, Martin, D-94081 Fürstenzell (DE); SEIKOWSKY, Axel, D-84347 Pfarrkirchen (DE); SELLEN, Martin, D-94496 Ortenburg (DE); WISSPEINTNER, Karl, D-94496 Ortenburg (DE)
(74) Vertreter: Naumann, Ulrich, Dr.-Ing.
(86) Internationale Anmeldenummer: DE9601867
(87) Internationale Veröffentlichungsnummer: WO9814751

(56) Entgegenhaltungen:
- EP-A- 0 078 096
- WO-A-91/15730
- DE-A- 19 511 939
- US-A- 3 358 225
- US-A- 3 884 076
- US-A- 4 339 714

## Beschreibung

Die Erfindung betrifft einen Sensor zur berührungslosen Dickenmessung an Folien, insbesondere an Blasfolien, mit einem Sensorkopf und einer Halterung für den Sensorkopf, wobei der Sensorkopf mindestens ein berührungslos, in Abstimmung auf die physikalischen Eigenschaften der Folie arbeitendes Sensorelement zur Dickenmessung umfaßt.

Sensoren zur berührungslosen Dickenmessung sind in den verschiedensten Ausführungen aus der Praxis bekannt. Beispielsweise wird die Dicke von Folienbändern während der Fertigung mit zwei einander gegenüberliegend angeordneten Wegmeßsensoren überwacht, indem das Folienband zwischen den Wegmeßsensoren geführt wird. Diese Sensoranordnung ist in der Praxis problematisch, da Wegmeßsensoren in der Regel innerhalb ihres Meßbereichs ein nichtlineares Verhalten zeigen. Durch Flattern des zu überwachenden Folienbandes, durch unterschiedliche Banddicken oder durch Bewegung des Bandes zu den Wegmeßsensoren hin oder von den Wegmeßsensoren weg treten Meßfehler auf, die auf das nichtlineare Verhalten der Wegmeßsensoren im Meßbereich zurückzuführen sind.

Für Dickenmessungen an Blasfolien ist die bekannte Sensoranordnung gänzlich ungeeignet, da sich eine Blasfolie nicht zwischen den beiden Wegmeßsensoren führen läßt. Daher wurden bislang in der Regel tastende Sensoranordnungen zur Dickenmessung an Blasfolien verwendet. Da die Dickenmessung aber in einem Stadium des Herstellungsverfahrens erfolgen soll, in dem das Folienmaterial noch weich und verformbar ist, hinterläßt eine tastende Dickenmessung immer Schleifspuren des Sensorkopfes, wenn sie nicht sogar zu Beschädigungen des Folienbandes und im Extremfalle zum Abreißen des Folienbandes führt.

Druckschrift US-A-3 884 076 beschreibt eine Meßvorrichtung, mit der sich die Dicke eines Produkts berührunglos bestimmen läßt, und entspricht dem ersten Teil des Anspruchs 1.

Der Erfindung liegt daher die Aufgabe zugrunde, einen Sensor zur Dickenmessung an Folien anzugeben, mit dem einerseits eine berührungslose Dickenmessung, insbesondere auch an Blasfolien, möglich ist und andererseits Meßfehler bedingt durch die Bewegung der Folie im Fertigungsprozeß weitgehend vermieden werden.

Der erfindungsgemäße Sensor löst die voranstehende Aufgabe durch die Merkmale des Patentanspruches 1. Danach ist der eingangs genannte Sensor derart ausgebildet, daß die Position des Sensorkopfes derart regelbar ist, daß der Sensorkopf während des gesamten Meßvorgangs in einem vorgebbaren, zumindest weitgehend konstanten Abstand zu der Folie gehalten ist, um eine definierte Relativlage zwischen dem Sensorkopf und der sich zufällig auf den Sensorkopf zu oder von diesem weg bewegenden Folie einzuhalten.

Erfindungsgemäß ist erkannt worden, daß insbesondere für Messungen an Folien während des Herstellungsprozesses berührungslos arbeitende Sensorelemente zu bevorzugen sind, da diese den Verformungsprozeß des Folienmaterials keinesfalls beeinflussen können. Es ist femer erkannt worden, daß die Wahl des Sensorelements bzw. eines geeigneten Meßverfahrens wesentlich von den physikalischen Eigenschaften des Folienmaterials abhängt. Schließlich ist noch erkannt worden, daß bei allen berührungslos arbeitenden Sensorelementen Voraussetzung für eine möglichst zuverlässige Meßwerterfassung ein definierter Abstand zwischen dem Sensorelement und dem Meßobjekt, hier der Folie, ist. Davon ausgehend ist erkannt worden, daß ein definierter Abstand zwischen Sensorkopf bzw. Sensorelement und der Folie nicht notwendigerweise eine Fixierung der Folie gegenüber dem Sensorkopf erfordert, sondern daß lediglich eine definierte Relativlage zwischen der Folie und dem Sensorkopf eingehalten werden muß. Erfindungsgemäß wird daher vorgeschlagen, die Position des Sensorkopfes dynamisch an die Lage der Folie anzupassen. Die Position des Sensorkopfes soll also nicht lediglich einmal auf die voraussichtliche Lage der Folie eingestellt werden sondern soll derart regelbar sein, daß der Sensorkopf während des gesamten Meßvorganges in einem vorgebbaren, zumindest weitgehend konstanten Abstand zu der Folie gehalten ist. Das heißt, erfindungsgemäß wird nicht ausschließlich die Folienlage fixiert sondern auch die Lage des Sensorkopfes auf die Bewegung der Folie abgestimmt.

In einer besonders vorteilhaften Ausführungsform des erfindungsgemäßen Sensors liefert das Sensorelement in einem kleinen Abstandsbereich zwischen Sensorkopf und Folie abstandsunabhängige Meßergebnisse. Auf diese Weise können kleinere Ungenauigkeiten bei der Positionsregelung des Sensorkopfes vernachlässigt werden. Auch ein gewisser Zeitversatz zwischen der Folienbewegung und der Bewegung des Sensorkopfes wirkt sich dann noch nicht negativ auf die Meßgenauigkeit des Sensors aus, wenn der Abstand zwischen Sensorkopf und Folie innerhalb des Abstandsbereichs der abstandsunabhängigen Meßergebnisse liegt.

Insbesondere zur Dickenmessung an Blasfolien haben sich kapazitiv arbeitende Sensorelemente bewährt. Blasfolien werden nämlich in der Regel aus einem Kunststoffmaterial hergestellt, welches als Dielektrikum die Kapazität einer Kondensatoranordnung beeinflußt. Im Gegensatz dazu bieten sich induktive Sensorelemente im Falle eines elektrisch leitfähigen Folienmaterials an. Derartige Materialien wechselwirken mit dem Feld einer Meßspule und beeinflussen dadurch die Induktivität der Meßspule. Berührungslose Dickenmessungen können auch mit optisch arbeitenden Sensorelementen durchgeführt werden, was dann eine entsprechende Meßanordnung und ein geeignetes Folienmaterial erfordert.

In einer vorteilhaften Ausgestaltung des erfindungsgemäßen Sensors ist der Sensorkopf in der Halterung verschiebbar gelagert. Dies ist insbesondere deshalb vorteilhaft, weil die Position des Sensorkopfes im wesentlichen nur senkrecht zur Transportrichtung der Folie geregelt werden muß. Bei entsprechender Orientierung der Halterung ist also lediglich eine eindimensionale Beweglichkeit des Sensorkopfes erforderlich, die sich am einfachsten in einer Linearverschiebung realisieren läßt.

Grundsätzlich sind ganz unterschiedliche Antriebsmittel zur Positionsregelung des Sensorkopfes denkbar.

Besonders vorteilhaft ist eine Positionsregelung mit Hilfe einer Gaszuführung im Sensorkopf und mindestens einer meßseitig angeordneten Austrittsöffnung. Über die Gaszuführung und die Austrittsöffnung wird Gas mit einem vorbestimmten Druck auf das Meßobjekt, nämlich die Folie, geleitet. Dabei überlagern sich zwei Effekte, so daß sich zwischen dem Sensorkopf und der Folie ein Luftpolster ausbildet. Einerseits wird die Folie aufgrund des Gasdrucks vom Sensorkopf weggedrückt, während die Folie andererseits zumindest im Randbereich des Sensorkopfes aufgrund des seitlich zwischem dem Sensorkopf und der Folie wegströmenden Gases und des dadurch entstehenden Unterdrucks angezogen wird. Wird nun der Gasdruck auf die Anordnung und geometrische Ausgestaltung der Austrittsöffnung abgestimmt sowie auf den Gegendruck der Folie, so bildet sich ein Luftpolster zwischen dem Sensorkopf und der Folie aus, dessen Dicke den Abstand zwischen dem Sensorkopf und der Folie bestimmt. Besonders vorteilhaft bei dieser Vorgehensweise ist, daß sich bei geeigneter Wahl der Betriebsparameter nach einem kurzen Einschwingvorgang ein annähemd konstanter Abstand zwischen Sensorkopf und Folie einstellt, der sich praktisch während des gesamten Meßvorgangs selbst einregelt.

Die Verwendung von Druckgas als Mittel zur Positionsregelung des Sensorkopfes hat außerdem noch mehrere positive Randeffekte. Zum einen dient das durch den Sensorkopf auf die Folie geleitete Gas zur Temperaturstabilisierung und Innenkühlung der Meßanordnung. Dadurch können auch temperaturbedingte Meßfehler weitgehend vermieden werden. Zum anderen kann das Gas je nach Zusammensetzung gegebenenfalls noch zur Weiterbehandlung des Folienmaterials verwendet werden.

Besonders einfach, kostengünstig und daher vorteilhaft ist es, wenn als Gas Druckluft verwendet wird. Denkbar wäre aber auch der Einsatz eines Schutzgases oder eines Gases, das sich günstig auf das Folienmaterial auswirkt.

Der selbstregulierende Effekt der voranstehend beschriebenen Sensoranordnung kann noch durch die spezielle konstruktive Ausgestaltung des Sensorkopfes positiv verstärkt werden. In diesem Zusammenhang ist es vorteilhaft, wenn die Meßseite des Sensorkopfes plattenförmig ausgebildet ist und mehrere Austrittsöffnungen vorzugsweise konzentrisch um die Position des Sensorelements, vorzugsweise in der Plattenmitte, angeordnet sind. In diesem Falle kann sich ein relativ großes Luftpolster im Bereich des Sensorelements ausbilden.

Der Ansaugeffekt zwischen der Platte und der Folie, kann dadurch reguliert werden, daß meßseitig in der Platte des Sensorkopfes Nuten bzw. Rillen ausgebildet sind, in die die Austrittsöffnungen münden. Ein derartiger Sensorkopf zeigt je nach der Orientierung der Nuten und Rillen ein unterschiedliches Positionsregelverhalten. Insgesamt läßt sich also der Abstand zwischen dem Sensorkopf und der Folie bei vorgegebenem Gegendruck der Folie in vorteilhafter Weise über den Gasdruck in Verbindung mit der Dimensionierung und Anordnung der Austrittsöffnungen sowie der Dimensionierung, Anordnung und Orientierung der Nuten oder Rillen regeln.

Als Gaszuführung bietet sich in einer vorteilhaften Ausgestaltung des erfindungsgemäßen Sensors ein endseitig am Sensorkopf angeordnetes Rohr an. Zusätzlich von Vorteil ist es, wenn der Sensorkopf über das Rohr verschiebbar in der Halterung gelagert ist. Im Hinblick auf eine Selbstregelung der Position des Sensorkopfes sollte die Lagerung des Sensorskopfes bzw. des Rohres möglichst reibungsarm sein. Eine bevorzugte Möglichkeit besteht darin, den Sensorkopf über Luftlager in der Halterung anzuordnen, so daß der Sensorkopf im wesentlichen reibungsfrei verschiebbar ist. Die Halterung könnte dazu ein Führungsrohr für das den Sensorkopf tragende Rohr umfassen, in dessen Wandung Durchtrittsöffnungen zur Einleitung von Luft ausgebildet sind. Das Rohr würde dann quasi auf einem Luftkissen innerhalb des Führungsrohres hin und her gleiten können.

Zusätzlich zu der Feineinstellung der Sensorkopfposition könnte die Halterung für den Sensorkopf eine Zustelleinrichtung zur Grobeinstellung der Position des Sensorkopfes und zur "Versteifung" der Halterung des Sensorkopfes umfassen. Die Versteifung soll eine Dämpfung des Systems bewirken, durch die eine hochfrequente Bewegung des Sensorkopfes unterdrückt wird. Eine solche Zustelleinrichtung könnte in vorteilhafter Weise einen geregelten Antrieb umfassen, wobei der Abstand zwischen Sensorkopf und Folie als Regelgröße dient. Um nun auch die Grobeinstellung der Position des Sensorkopfes soweit wie möglich zu automatisieren, könnte der erfindungsgemäße Sensor zur berührungslosen Dickenmessung einen zusätzlichen Abstandssensor umfassen. Dieser könnte beispielsweise kapazitiv, induktiv oder nach dem Ultraschall-Meßprinzip arbeiten. Denkbar wären aber auch andere Meßverfahren in Abhängigkeit von dem Folienmaterial, wie z.B. das optische Meßprinzip. Da der Abstandssensor der Grobeinstellung des Sensorkopfes dienen soll, ist es vorteilhaft, wenn der Abstandssensor auch selbst am Sensorkopf angeordnet ist.

Genauso wie ganz unterschiedliche Antriebsmittel zur Positionsregelung des Sensorkopfes eingesetzt werden können, sind auch unterschiedliche Antriebsformen für die Zustelleinrichtung denkbar.

Der Antrieb der Zustelleinrichtung könnte bspw. pneumatisch erfolgen, was insbesondere in Verbindung mit einer Positionsregelung des Sensorkopfes mit Hilfe einer Gaszuführung im Sensorkopf vorteilhaft ist. Im Falle der in diesem Zusammenhang bereits beschriebenen Sensoranordnung könnte dazu in dem Führungsrohr eine Druckkammer ausgebildet sein, durch die das den Sensorkopf tragende Rohr geführt ist. Das Rohr könnte mit einer im Bereich der Druckkammer angeordneten und senkrecht zur Bewegungsrichtung des Sensorkopfes orientierten Druckplatte versehen sein, so daß die Druckplatte die Druckkammer in zwei Teilkammem aufteilt. Ist nun jede der Teilkammem mit einem Anschluß an eine Gasleitung versehen, welche in den Sensorkopf geführt ist, so wirken sich Veränderungen der Druckverhältnisse vor dem Sensorkopf und damit Änderungen des Abstandes zwischen dem Sensorkopf und der Folie direkt auf die Druckverhältnisse in den beiden Teilkammem der Druckkammer aus. Durch entsprechenden Anschluß der beiden Teilkammem an die Gasleitungen kann einer hochfrequenten Bewegung des Sensorkopfes entgegengewirkt werden und also eine Art Dämpfung und Stabilisierung der Lage des Sensorkopfes erzielt werden.

Der Antrieb der Zustelleinrichtung könnte aber auch elektrisch oder magnetisch erfolgen. Als Antrieb könnte beispielsweise ein Piezomotor dienen oder auch im Falle einer mechanischen Variante eine Spindel.

Die Dämpfung der Halterung des Sensorkopfes bzw. das Unterdrücken von sehr hochfrequenten Bewegungen des Sensorkopfes ist insbesondere dann wichtig, wenn Leitungsverbindungen zur Energieeinspeisung in den Sensorkopf und/oder zur Signalübertragung zwischen dem Sensorkopf und einer Auswerte-/Regeieinheit vorgesehen sind. In der Praxis können diese Leitungsverbindungen durch sehr dünne Koaxialkabel realisiert sein, die die Bewegung des Sensorkopfes möglichst wenig beeinträchtigen, aber dennoch eine mechanische Kopplung zwischen dem verschiebbar gelagerten Sensorkopf und einem stationären Sensorteil darstellen. Solche Leitungsverbindungen sind besonders anfällig gegen hochfrequente Bewegungen. Bei einer Dämpfung des Systems, d.h. durch Unterdrückung von hochfrequenten Bewegungen, läßt sich diese Schwachstelle des erfindungsgemäßen Sensors jedoch weitgehend beheben.

In einer besonders vorteilhaften Variante des erfindungsgemäßen Sensors erfolgt die Energieeinspeisung in den Sensorkopf berührungslos, d.h. ohne eine entsprechende Leitungsverbindung zwischen dem beweglich gelagerten Sensorkopf und einem stationären Sensorteil. Die Energieeinspeisung könnte dazu induktiv nach dem Transformatorprinzip erfolgen. Wenn außerdem auch die Signalübertragung zwischen dem Sensorkopf und einer Auswerte-/Regeleinheit berührungslos erfolgt, besteht keine mechanische Kopplung zwischen dem Sensorkopf und dem stationären Sensorteil mehr, so daß die Bewegung des Sensorkopfes ungehindert erfolgen kann. Ein Leitungsbruch aufgrund hochfrequenter Bewegungen des Sensorkopfes kann hier auch nicht mehr auftreten. Die Signalübertragung zwischen dem Sensorkopf und der Auswerte-/Regeleinheit könnte optisch, induktiv oder auch kapazitiv erfolgen. Besonders vorteilhaft ist es, eine Sender/Empfänger-Einrichtung zur Signalübertragung einzusetzen. Verfügt diese Sender/Empfänger-Einrichtung über eine entsprechende Modulationseinrichtung, so lassen sich die aus der Signalübertragung bekannten Signalmodulationsverfahren, Puls-Code-Modulation, Frequenzmodulation und Amplitudenmodulation, auf die zu übertragenden Signale anwenden, so daß sich auch eine gewisse Fehlerunanfälligkeit bei der Signalübertragung gewährleisten läßt. In jedem Falle ist es vorteilhaft, die Meßsignale vor der Signalübertragung vom Sensorkopf zu der Auswerte-/Regeleinheit mit Hilfe eines Vorverstärkers zu verstärken.

Hinsichtlich der im Rahmen des erfindungsgemäßen Sensors eingesetzten Auwerte/Regeleinheit sei noch angemerkt, daß diese auch teilweise in den Sensorkopf integriert sein kann. Zum Beispiel kann im Sensorkopf bereits ein Schwingkreis als Teil der Auswerteschaltung angeordnet sein. Mit Hilfe der Auswerte-/Regeleinheit sollen in erster Linie die Meßsignale ausgewertet werden, d.h. die Dicke der Folie bestimmt werden. Zusätzlich läßt sich aber auch der Abstand zwischen dem Sensorkopf und der Folie bestimmen und zur Regelung des Antriebs der Zustelleinrichtung einsetzen. Die Auswerte-/Regeleinheit könnte zusätzlich noch mit einer Elektronik ausgestattet sein zur Überwachung des Einbauzustandes des Sensorkopfes, zur Funktionskontrolle des Sensors insgesamt, bspw. zur Kontrolle des Gasdrucks, und zur Zuordnung der Dickenmeßwerte zum Meßort, d.h. zur Position an der Folie. Dazu könnten zusätzlich zwei Timer vorgesehen sein, die zusätzlich zu den Dickenmeßwerten auch Zeitinformationen erfassen, über die sich dann der Meßort bestimmen läßt.

Es gibt nun verschiedene Möglichkeiten, die Lehre der vorliegenden Erfindung in vorteilhafter Weise auszugestalten und weiterzubilden. Dazu ist einerseits auf die dem Patentanspruch 1 nachgeordneten Ansprüche, andererseits auf die nachfolgende Erläuterung eines Ausführungsbeispiels der Erfindung anhand der Zeichnung zu verweisen. In Verbindung mit der Erläuterung des bevorzugten Ausführungsbeispiels der Erfindung anhand der Zeichnung werden auch im allgemeinen bevorzugte Ausgestaltungen und Weiterbildungen der Lehre erläutert.

In der Zeichnung zeigt
Figur 1 einen Längsschnitt durch einen erfindungsgemäßen Sensor zur berührungslosen Dickenmessung an Folien,
Figur 2 einen erfindungsgemäßen Sensor mit einer pneumatisch arbeitenden Zustelleinrichtung,
Figur 3 einen weiteren erfindungsgemäßen Sensor mit einer einen Aktuator umfassenden Zustelleinrichtung und
Figur 4 einen erfindungsgemäßen Sensor, bei dem die Energieeinspeisung und Signalübertragung berührungslos erfolgt.

Der Sensor 1 ist insbesondere geeignet zur berührungslosen Dickenmessung an Blasfolien. Zur Herstellung derartiger Folien wird das erwärmte Ausgangsmaterial extrudiert und gleichzeitig geblasen. Die Dickenmessung mit Hilfe des erfindungsgemäßen Sensors 1 erfolgt während des Herstellungsverfahrens zu einem Zeitpunkt, an dem das Folienmaterial 2 noch nicht vollständig ausgekühlt und erhärtet ist. Derartige Dickenmessungen werden zur Qualitätskontrolle während des Herstellungsprozesses vorgenommen, um frühzeitig material- oder verfahrensbedingte Produktionsfehler zu erkennen und Gegenmaßnahmen ergreifen zu können. Die berührungslose Dickenmessung ermöglicht hier eine Qualitätskontrolle, ohne daß die noch verformbare Folie in irgendeiner Weise beeinträchtig wird.

Der hier dargestellte Sensor 1 umfaßt einen Sensorkopf 3 und eine Halterung 4 für den Sensorkopf.

Erfindungsgemäß umfaßt der Sensorkopf 3 mindestens ein hier nicht dargestelltes berührungslos, in Abstimmung auf die physikalischen Eigenschaften der Folie 2 arbeitendes Sensorelement zur Dickenmessung. Die Position des Sensorkopfes 3 ist derart regelbar, daß der Sensorkopf 3 während des gesamten Meßvorganges in einem vorgebbaren, zumindest weitgehend konstanten Abstand zu der Folie 2 gehalten ist.

Prinzipiell kann im Rahmen des erfindungsgemäßen Sensors jedes zur Dickenmessung geeignete, berührungslos arbeitende Sensorelement eingesetzt werden. Welche Meßmethode letztlich angewendet wird, hängt entscheidend von den physikalischen Eigenschaften der Folie 2 ab. Vorteilhaft ist es, wenn das Sensorelement zumindest in einem kleinen Abstandsbereich zwischen Sensorkopf 3 und Folie 2 abstandsunabhängige Meßergebnisse liefert und der Abstand zwischen Sensorkopf 3 und Folie 2 auch innerhalb des Abstandsbereiches der abstandsunabhängigen Meßergebnisse liegt. Dann nämlich lassen sich vergleichsweise fehlerunanfällige Messungen durchführen.

Im vorliegenden Fall der Dickenmessung an Blasfolien, die in der Regel aus einem als Dielektrikum wirkenden Kunststoff hergestellt werden, umfaßt der erfindungsgemäße Sensor 1 ein kapazitiv arbeitendes Sensorelement zur Dickenmessung.

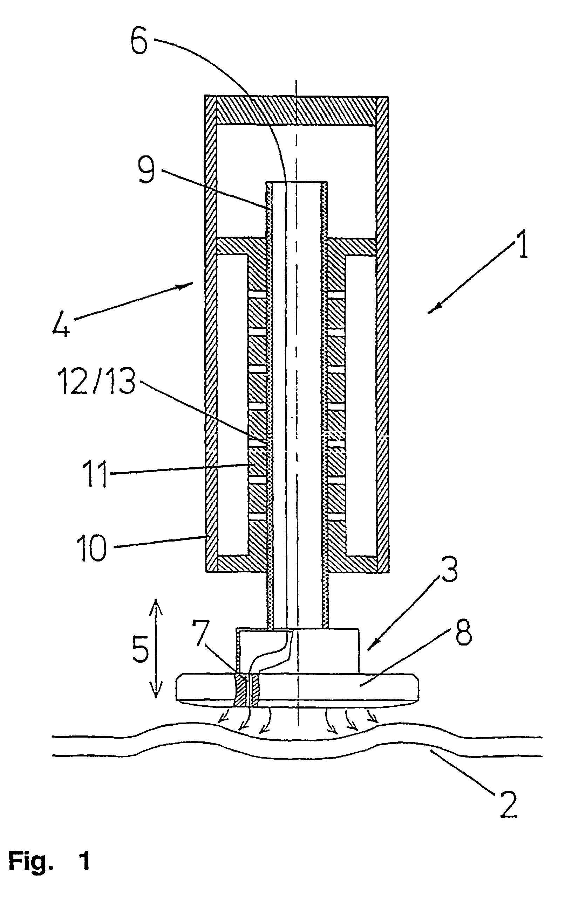

Der Sensorkopf 3 des hier dargestellten Sensors 1 ist in der Halterung 4 verschiebbar gelagert, was durch den Doppelpfeil 5 angedeutet ist. Der Sensorkopf 3 kann danach senkrecht zur Folie 2 bzw. zur Transportrichtung der Folie 2 bewegt werden. Als Antrieb dieser Bewegung und also als Mittel zur Positionsregelung weist der Sensorkopf 3 eine Gaszuführung 6 und meßseitig mehrere Austrittsöffungen 7 auf. Im dargestellten Ausführungsbeispiel ist die Meßseite des Sensorkopfes 3 plattenförmig (8) ausgebildet. Von den Austrittsöffnungen 7 ist lediglich beispielhaft eine dargestellt. Insgesamt sind die Austrittsöffnungen 7 konzentrisch in einem bestimmten Abstand um die Plattenmitte angeordnet, wo das Sensorelement lokalisiert ist. Vorteilhafterweise münden die Austrittsöffnungen 7 in meßseitig in der Platte 8 ausgebildeten Nuten oder Rillen, die Figur 1 nicht zu entnehmen sind.

In dem dargestellten Ausführungsbeispiel wird Druckluft durch den Sensorkopf 3 auf die Folie 2 geleitet. Bei geeigneter Abstimmung von Druck, Anzahl, Anordnung und Geometrie der Austrittsöffnungen 7 und Nuten und Rillen sowie des Gegendrucks der Folie 2, die an dem Sensorkopf 3 vorbeigeführt wird, bildet sich ein Luftkissen im Bereich des Sensorelements, also der Plattenmitte, zwischen Sensorkopf 3 und Folie 2 aus. Dies ist einerseits auf den durch die Druckluft auf die Folie 2 ausgeübten Druck und andererseits auf den Unterdruck zwischen dem Sensorkopf 3 bzw. der Platte 8 und der Folie 2 in den Randbereichen der Platte 8 zurückzuführen. Dadurch nämlich wird die Relativlage zwischen dem Sensorkopf und der Folie 2 stabilisiert. Nach einer kurzen Einschwingphase besteht dann ein konstanter Abstand zwischen Sensorkopf 3 und Folie 2. Dies gilt auch während des Transports der Folie 2 bei etwaig auftretendem Flattern der Folie 2. Dadurch ist eine berührungslose und abstandsunabhängige Dickenmessung auch an der laufenden, noch weichen elastischen Folie 2 möglich.

Die aus dem Sensorkopf 3 austretende Druckluft hat zusätzlich noch einen Kühleffekt, so daß die Dickenmessungen in einem konstanten Temperaturbereich durchgeführt werden und also auch Meßfehler bedingt durch Temperaturschwankungen weitgehend vermieden werden.

In dem in Figur 1 dargestellten Ausführungsbeispiel eines erfindungsgemäßen Sensors 1 ist der Sensorkopf 3 endseitig an einem Rohr 9 angeordnet, das gleichzeitig als Gaszuführung 6 für den Sensorkopf 3 dient. Der Sensorkopf 3 ist über das Rohr 9 in der Halterung 4 verschiebbar gelagert. Genauer gesagt umfaßt die Halterung 4 ein Gehäuse 10 mit einem Führungsrohr 11 für das Rohr 9. In der Wandung des Führungsrohrs 11 sind Durchtrittsöffnungen 12 zur Einleitung von Luft ausgebildet, die letztlich als Luftlager 13 für das Rohr 9 und also für den Sensorkopf 3 dienen. Über die Luftlager 13 ist der Sensorkopf 3 im wesentlichen reibungsfrei in der Halterung 4 verschiebbar gelagert.

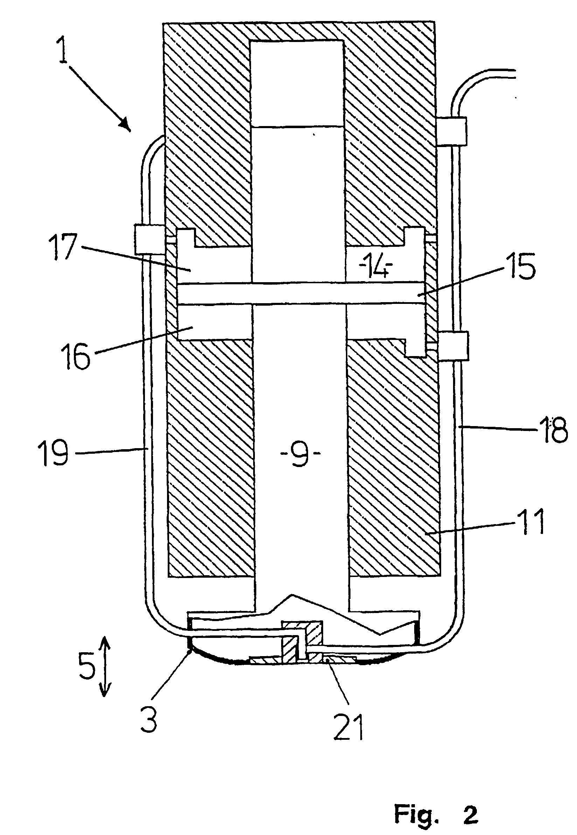

Die Halterung des Sensors 1 könnte zusätzlich noch eine Zustelleinrichtung zur Grobeinstellung und Stabilisierung der Position des Sensorkopfes umfassen. In Figur 2 ist ein solcher Sensor 1 mit einer pneumatisch arbeitenden Zustelleinrichtung dargestellt. Der Sensorkopf 3 ist hier ebenfalls verschiebbar gelagert, was durch den Doppelpfeil 5 angedeutet ist. Dazu ist der Meßkopf 3 an einem Rohr 9 befestigt, welches in einem Führungsrohr 11 verschiebbar gelagert ist. In dem Führungsrohr 11 ist eine Druckkammer 14 ausgebildet, durch die das Rohr 9 geführt ist. Im Bereich der Druckkammer 14 ist eine Druckplatte 15 an dem Rohr 9 angeordnet, die so dimensioniert ist, daß sie die Druckkammer 14 in zwei Teilkammem 16 und 17 unterteilt. Jede der beiden Teilkammern 16 und 17 ist an eine Gasleitung 18, 19 angeschlossen, welche wiederum in den Sensorkopf 3 geführt sind. Dadurch wirkt sich der Staudruck, der sich zwischen dem Sensorkopf 3 und der in Figur 2 nicht dargestellten Folie aufbaut, direkt auf die in den Teilkammem 16 und 17 herrschenden Drücke aus. Der Staudruck wiederum ist abhängig vom Abstand zwischen dem Meßkopf 3 und der Folie. Eine Veränderung des Abstandes resultiert in einer Druckdifferenz zwischen den in den beiden Teilkammem 16 und 17 herrschenden Drücken. Dadurch wird eine Kraft auf die Druckplatte 15 und somit auf das Rohr 9 ausgeübt. Bei entsprechendem Anschluß der Teilkammern 16 und 17 an die Gasleitungen 18 und 19 lassen sich so hochfrequente Bewegungen des Meßkopfes 3 unterdrücken.

Im Zusammenhang mit Figur 2 sei angemerkt, daß es sich hier lediglich um eine schematische Darstellung handelt, die verdeutlichen soll, daß eine Grobeinstellung der Position des Sensorkopfes 3 und auch eine Dämpfung der Bewegung des Sensorkopfes 3 auf pneumatischem Wege erreichbar ist.

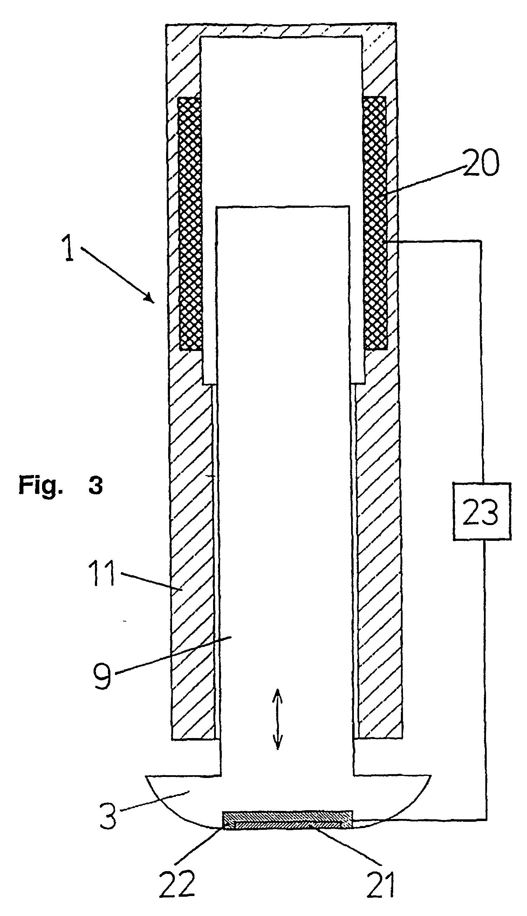

Figur 3 soll veranschaulichen, daß sich die Position des Sensorkopfes 3 auch mit Hilfe einer einen Aktuator umfassenden Zustelleinrichtung stabilisieren läßt. Dazu ist an der Halterung 4 des Sensorkopfes 3 ein Aktuator 20 angeordnet, über den sich das Rohr 9 innerhalb des Führungsrohrs 11 verschieben läßt. Bei dem Aktuator 20 kann es sich um einen elektrisch oder auch magnetisch betriebenen Antrieb handeln. In Frage kommt beispielsweise auch ein Piezomotor oder eine Spindel als mechanischer Antrieb. Zur Regelung der Position des Sensorkopfes 3 umfaßt der Sensor 1 zusätzlich zu dem Sensorelement 21, mit dem die Dicke der Folie erfaßt wird, noch einen Abstandssensor 22, mit dem der Abstand zwischen dem Sensorkopf 3 und der Folie ermittelt wird. Dieser Abstandswert dient als Regelgröße für den Aktuator 20, was in Figur 3 durch einen PID-Regler, proportional integrierter und differenzierender Regler 23 angedeutet ist.

In Verbindung mit Figur 3 sei noch angemerkt, daß hier das Sensor im Sensor-System realisiert ist, indem nämlich der Abstandssensor 22 um das die Dicke der Folie erfassende Sensorelement 21 angeordnet ist. Auf diese Weise wird sichergestellt, daß mit dem Abstandssensor 22 auch tatsächlich der für die Messung relevante Abstand zwischen dem Sensorelement 21 und der Folie bestimmt wird.

Mit Hilfe der in Verbindung mit den Figuren 2 und 3 erläuterten Zustelleinrichtungen lassen sich - wie bereits mehrfach erwähnt - sowohl eine Grobeinstellung der Position des Sensorkopfes als auch eine Stabilisierung dieser Position, d.h. eine Unterdrückung von hochfrequenten Bewegungen des Sensorkopfes, durchführen. Dies ist insbesondere dann wichtig, wenn die Energieeinspeisung und/oder die Signalübertragung vom Sensorkopf zu einer Auswerte-/Regelschaltung über Leitungsverbindungen erfolgt. Derartige Leitungsverbindungen müssen so dünn und filigran sein, daß sie die Bewegung des Sensorkopfes möglichst wenig beeinträchtigen. Dadurch weisen sie aber nur eine sehr geringe mechanische Stabilität auf, so daß sie insbesondere durch hochfrequente Bewegungen schnell brechen.

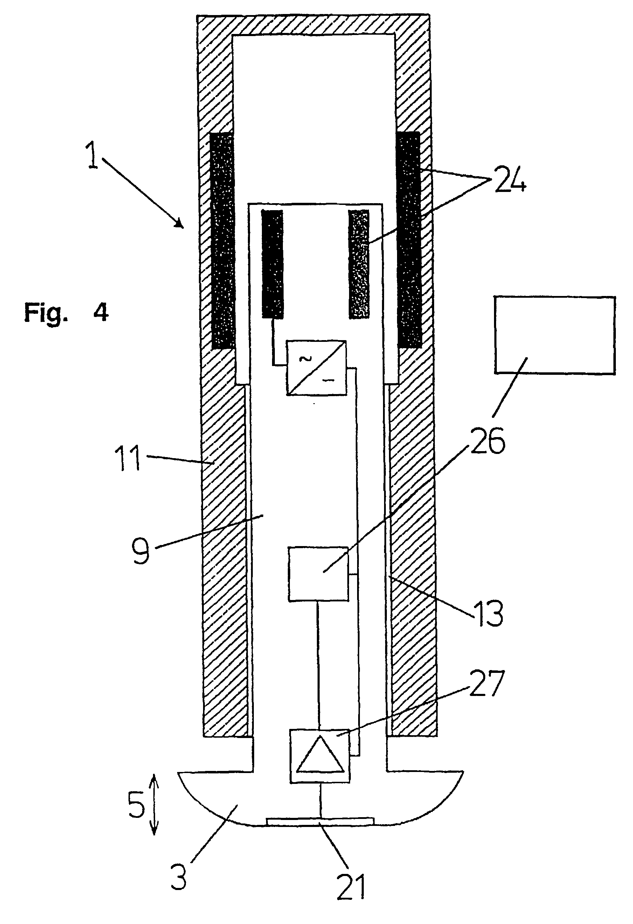

In Figur 4 ist nun eine Ausführungsform des erfindungsgemäßen Sensors 1 dargestellt, bei dem die Energieeinspeisung berührungslos, nämlich induktiv nach dem Transformatorprinzip erfolgt. Dazu ist sowohl im Bereich des Führungsrohrs 11 als auch im entsprechenden Bereich des Rohres 9 jeweils eine Spule 24 angeordnet, über die die Energieeinspeisung in den Sensorkopf 3 erfolgt. Die Übertragung der Meßsignale vom Sensorkopf 3 zu einer Auswerte-/Regeleinrichtung 25, die extern, beispielsweise im Gehäuse des Sensors 1 angeordnet ist, erfolgt hier ebenfalls berührungslos mit Hilfe einer Sender/Empfänger-Einrichtung 26. Die Signalübertragung könnte beispielsweise aber auch auf optischem Wege erfolgen. Im Bereich des Sensorkopfes 3 bzw. dem sich anschließenden Rohr 9 ist ein Teil der Auswerteschaltung angeordnet, der einen Vorverstärker 27 umfaßt. Das verstärkte Signal wird dann an die Auswerte-/Regelschaltung 25 gesendet. Um die Fehleranfälligkeit einer solchen Signalübertragung zu verringern kann vorher auch noch eine Modulation des Meßsignals erfolgen, beispielsweise eine Puls-Code-Modulation, eine Frequenzmodulation oder auch eine Amplitudenmodulation.

Zusammenfassend sei an dieser Stelle nochmals hervorgehoben, daß bei den hier dargestellten Ausführungsbeispielen von erfindungsgemäßen Sensoren zur berührungslosen Dickenmessung an Folien in der Regel zunächst eine Grobeinstellung des Abstandes zwischen Sensorkopf und Folie mit Hilfe eines zusätzlichen Abstandssensors vorgenommen wird. Die Feineinstellung des Abstandes zwischen Sensorkopf und Folie erfolgt automatisch, indem mit Hilfe von Druckluft ein kleiner Luftspalt bzw. ein Luftpolster zwischen Sensorkopf und Folie erzeugt wird, dessen Breite vorzugsweise im Bereich der abstandsunabhängigen Meßergebnisse des Sensorelements zur Dickenmessung liegt. Zur Einstellung des Luftspalts wird Druckluft über den Sensorkopf auf die Folie geleitet, so daß sich am Rande des Sensorkopfes ein Unterdruck ausbildet und die Folie dadurch angesaugt wird, jedoch ohne den Sensorkopf selbst zu berühren. Daneben ist der Sensorkopf noch reibungsfrei innerhalb der Halterung des Sensors gelagert, so daß nicht nur die Position der Folie selbst modifiziert wird sondern auch die Position des Sensorkopfes. Bei dem erfindungsgemäßen Sensor wird der Abstand zwischen Sensorkopf und Folie also durch Regelung der Relativlage zwischen Sensorkopf und Folie konstant gehalten.

Hinsichtlich weiterer in der einzigen Figur nicht gezeigter Merkmale wird auf den allgemeinen Teil der Beschreibung verwiesen.

Abschließend sei hervorgehoben, daß die erfindungsgemäße Lehre nicht nur zur Dickenmessung an Blasfolien geeignet ist, sondern sich generell zur Dickenmessung an Folien aus beliebigem Material eignet.

## Patentansprüche

1. Sensor zur berührungslosen Dickenmessung an Folien (2), insbesondere an Blasfolien,
- mit einem Sensorkopf (3), wobei der Sensorkopf (3) mindestens ein berührungslos, in Abstimmung auf die physikalischen Eigenschaften der Folie (2) arbeitendes Sensorelement zur Dickenmessung umfaßt, und
- mit einer Halterung (4) für den Sensorkopf (3), die so ausgebildet ist, daß die Position des Sensorkopfes (3) regelbar ist, wobei der Sensorkopf (3) als Mittel zur Positionsregelung eine Gaszuführung (6) und meßseitig mindestens eine Austrittsöffnung (7) aufweist,
**dadurch gekennzeichnet, daß** die Feineinstellung des Abstandes zwischen Sensorkopf (3) und Folie (2) automatisch durch den über die Gaszuführung (6) und die Austrittsöffnung (7) auf die Folie (2) geleiteten Gasstrom erfolgt, über den einerseits die Position des Sensorkopfes (3) geregelt wird und andererseits im Randbereich des Sensorkopfes (3) ein Unterdruck erzeugt wird, durch den die Folie (2) angesaugt wird, ohne den Sensorkopf (3) selbst zu berühren, so daß während des gesamten Meßvorgangs eine vorgebbare, definierte, zumindest weitgehend konstante Relativlage zwischen dem Sensorkopf (3) und der sich zufällig auf den Sensorkopf (3) zu oder von diesem weg bewegenden Folie (2) eingehalten ist.

2. Sensor nach Anspruch 1, **dadurch gekennzeichnet, daß** das Sensorelement zumindest in einem kleinen Abstandsbereich zwischen Sensorkopf (3) und Folie (2) abstandsunabhängige Meßergebnisse liefert, wobei der Abstand zwischen Sensorkopf (3) und Folie (2) innerhalb des Abstandsbereichs der abstandsunabhängigen Meßergebnisse liegen kann.

3. Sensor nach Anspruch 1 oder 2, **dadurch gekennzeichnet, daß** das Sensorelement kapazitiv oder induktiv arbeitet.

4. Sensor nach einem der Ansprüche 1 bis 3, **dadurch gekennzeichnet, daß** der Sensorkopf (3) in der Halterung (4) verschiebbar gelagert ist.

5. Sensor nach einem der Ansprüche 1 bis 4, **dadurch gekennzeichnet, daß** die Gaszuführung (6) durch eine Druckluftzuführung gebildet ist.

6. Sensor nach Anspruch 5, **dadurch gekennzeichnet, daß** die Meßseite des Sensorkopfes (3) plattenförmig ausgebildet ist und daß mehrere Austrittsöffnungen (7) vorzugsweise konzentrisch um die Position des Sensorelements vorzugsweise in der Plattenmitte angeordnet sind, wobei die Austrittsöffnungen (7) vorzugsweise in meßseitig ausgebildete Nuten oder Rillen der Platte (8) münden.

7. Sensor nach Anspruch 5 oder 6, **dadurch gekennzeichnet, daß** der Abstand zwischen dem Sensorkopf (3) und der Folie (2) über den Gasdruck in Verbindung mit der Dimensionierung und Anordnung der Austrittsöffnung (7) sowie ggf. der Nuten oder Rillen regelbar ist.

8. Sensor nach einem der Ansprüche 5 bis 7, **dadurch gekennzeichnet, daß** der Sensorkopf (3) endseitig an einem Rohr (9) angeordnet ist und daß die Gaszuführung (6) durch das Rohr (9) geführt ist, wobei das Rohr (9) mit dem Sensorkopf (3) über Luftlager (13) im wesentlichen reibungsfrei in der Halterung (4) verschiebbar ist und/oder wobei die Halterung (4) ein Führungsrohr (11) für das Rohr (9) umfaßt und daß die Wandung des Führungsrohrs (11) Durchtrittsöffnungen (12) zur Einleitung von Luft aufweist.

9. Sensor nach einem der Ansprüche 1 bis 8, **dadurch gekennzeichnet, daß** die Halterung eine Zustelleinrichtung zur Grobeinstellung der Position des Sensorkopfes (3) umfaßt, wobei die Zustelleinrichtung einen geregelten Antrieb umfassen kann, wobei ein Abstandssensor (22) für die Grobeinstellung der Position des Sensorkopfes (3) vorgesehen sein kann, der kapazitiv, induktiv, optisch oder nach dem Ultraschall-Meßprinzip arbeitet und wobei der Abstandssensor (22) am Sensorkopf (3) angeordnet ist.

10. Sensor nach Anspruch 9, **dadurch gekennzeichnet, daß** der Antrieb der Zustelleinrichtung pneumatisch, elektrisch oder magnetisch erfolgt.

11. Sensor nach Anspruch 8 und nach Anspruch 10, **dadurch gekennzeichnet, daß** in dem Führungsrohr (11) eine Druckkammer (14) ausgebildet ist, daß das Rohr (9) durch die Druckkammer (14) geführt ist und im Bereich der Druckkammer (14) mit einer senkrecht zur Bewegungsrichtung des Sensorkopfes (3) orientierten Druckplatte (15) versehen ist, daß die Druckplatte (15) die Druckkammer (14) in zwei Teilkammem (16, 17) aufteilt, daß jede Teilkammern (16, 17) einen Anschluß an eine Gasleitung (18, 19) aufweist und daß die Gasleitung (18, 19) in den Sensorkopf (3) geführt ist.

12. Sensor nach Anspruch 10 oder 11, **dadurch gekennzeichnet, daß** ein Piezomotor oder eine Spindel als Antrieb dient.

13. Sensor nach einem der Ansprüche 1 bis 12, **dadurch gekennzeichnet, daß** Leitungsverbindungen zur Energieeinspeisung in den Sensorkopf und/oder Signalübertragung zwischen dem Sensorkopf und einer Auswerte-/Regeleinheit vorgesehen sind.

14. Sensor nach einem der Ansprüche 1 bis 12, **dadurch gekennzeichnet, daß** die Energieeinspeisung in den Sensorkopf berührungslos erfolgt, wobei die Energieeinspeisung induktiv nach dem Transformatorprinzip erfolgt.

15. Sensor nach einem der Ansprüche 1 bis 14, **dadurch gekennzeichnet, daß** die Signalübertragung zwischen dem Sensorkopf und einer Auswerte-/Regeleinheit berührungslos erfolgt, wobei die Signalübertragung optisch, induktiv oder kapazitiv erfolgt, wobei eine Sender/Empfänger-Einrichtung (26) zur Signalübertragung vorgesehen sein kann und wobei die Sender/Empfänger-Einrichtung (26) vorzugsweise eine Modulationseinrichtung zur Puls-Code-Modulation, Frequenzmodulation oder Amplitudenmodulation der Signale umfaßt.

16. Sensor nach Anspruch 15, **dadurch gekennzeichnet, daß** ein Vorverstärker (27) zur Verstärkung des Meßsignals vor der Signalübertragung vorgesehen ist.

## Claims

1. Sensor for the contactless thickness measurement of films (2), in particular blown films,
- having a sensor head (3), the sensor head (3) comprising at least one sensor element for measuring thicknesses which functions contactlessly in accordance with the physical properties of the film (2), and
- having a holding element (4) for the sensor head (3) which is of such a form that the position of the sensor head (3) is adjustable, the sensor head (3) having, as the means for position adjustment, a gas supply line (6) and, at the measurement side, at least one outlet opening (7),
**characterised in that** the fine adjustment of the spacing between the sensor head (3) and the film (2) is effected automatically by the gas flow, which is directed via the gas supply line (6) and the outlet opening (7) towards the film (2) and by means of which, on the one hand, the position of the sensor head (3) is adjusted and, on the other hand, a reduced pressure is generated in the edge region of the sensor head (3), by means of which reduced pressure the film (2) is drawn up without coming into contact with the sensor head (3) itself so that during the entire measurement operation a predeterminable, defined relative position, which is at least substantially constant, is maintained between the sensor head (3) and the film (2) which may move at random towards or away from the sensor head (3).

2. Sensor according to claim 1, **characterised in that** the sensor element supplies measurement results which are spacing-independent at least within a small spacing range between the sensor head (3) and the film (2), it being possible for the spacing between the sensor head (3) and the film (2) to be located within the spacing range of the spacing-independent measurement results.

3. Sensor according to claim 1 or claim 2, **characterised in that** the sensor element functions capacitatively or inductively.

4. Sensor according to any one of claims 1 to 3, **characterised in that** the sensor head (3) is arranged for displacement in the holding element (4).

5. Sensor according to any one of claims 1 to 4, **characterised in that** the gas supply line (6) comprises a compressed air supply line.

6. Sensor according to claim 5, **characterised in that** the measurement side of the sensor head (3) is plate-shaped and **in that** several outlet openings (7) are arranged preferably concentrically about the position of the sensor element, preferably in the centre of the plate, the outlet openings (7) preferably opening into channels or grooves of the plate (8) which are arranged on the measurement side.

7. Sensor according to claim 5 or claim 6, **characterised in that** the spacing between the sensor head (3) and the film (2) can be adjusted by means of the gas pressure in conjunction with the dimensioning and arrangement of the outlet opening (7) and, optionally, of the channels or grooves.

8. Sensor according to any one of claims 5 to 7, **characterised in that** the sensor head (3) is arranged at the end face of a tube (9) and **in that** the gas supply line (6) is guided through the tube (9), the tube (9) having the sensor head (3) being displaceable in a substantially frictionless manner in the holding element (4) by means of air bearings (13), and/or the holding element (4) comprising a guide tube (11) for the tube (9) and **in that** the wall of the guide tube (11) has through-openings (12) for the introduction of air.

9. Sensor according to any one of claims 1 to 8, **characterised in that** the holding element comprises a feed device for the coarse adjustment of the position of the sensor head (3), it being possible for the feed device to comprise a variable-speed drive, and for a spacing sensor (22) to be provided for the coarse adjustment of the position of the sensor head (3), which spacing sensor (22) functions capacitatively, inductively, optically or according to the ultrasound measurement principle, and the spacing sensor (22) being arranged on the sensor head (3).

10. Sensor according to claim 9, **characterised in that** the drive of the feed device is effected pneumatically, electrically or magnetically.

11. Sensor according to claim 8 and claim 10, **characterised in that** a pressure chamber (14) is arranged in the guide tube (11), **in that** the tube (9) is guided through the pressure chamber (14) and is provided in the region of the pressure chamber (14) with a pressure plate (15), which is orientated perpendicularly to the movement direction of the sensor head (3), **in that** the pressure plate (15) divides the pressure chamber (14) into two partial chambers (16, 17), **in that** each partial chamber (16, 17) has a connection to a gas line (18, 19) and **in that** the gas line (18, 19) is guided into the sensor head (3).

12. Sensor according to claim 10 or claim 11, **characterised in that** a piezo-electric motor or a spindle serves as the drive.

13. Sensor according to any one of claims 1 to 12, **characterised in that** line connections are provided for supplying energy to the sensor head and/or transmitting signals between the sensor head and an evaluation/control unit.

14. Sensor according to any one of claims 1 to 12, **characterised in that** the supply of energy to the sensor head is effected in a contactless manner, the energy supply being effected inductively according to the transformer principle.

15. Sensor according to any one of claims 1 to 14, **characterised in that** the transmission of signals between the sensor head and an evaluation/control unit is effected in a contactless manner, the signal transmission being effected optically, inductively or capacitatively, it being possible for a transmission/receiving device (26) to be provided for transmitting signals and the transmission/receiving device (26) preferably comprising a modulation device for the pulse-code modulation, frequency modulation or amplitude modulation of the signals.

16. Sensor according to claim 15, **characterised in that** a pre-amplifier (27) is provided to amplify the measurement signal before the signal transmission.

## Revendications

1. Capteur pour mesurer sans contact l'épaisseur de feuilles (2), en particulier de lamelles de soufflage,
- avec une tête de capteur (3), la tête de capteur (3) comprenant au moins un élément de capteur pour mesurer l'épaisseur, travaillant en accord avec les propriétés physiques de la feuille (2) et
- avec une fixation (4) pour la tête de capteur (3) qui est réalisée de telle manière que la tête de capteur (3) est réglable, la tête de capteur (3) présentant, en tant que moyen de réglage de position, une amenée de gaz (6) et, du côté mesure, au moins une ouverture de sortie (7),
**caractérisé en ce que** le réglage fin de la distance entre la tête de capteur (3) et la feuille (2) est effectué automatiquement par le courant de gaz guidé sur la feuille (2) par l'amenée de gaz (6) et l'ouverture de sortie (7), par lequel d'une part la position de la tête de capteur (3) est réglée et d'autre part une dépression est générée dans la zone de bord de la tête du capteur (3) par laquelle la feuille (2) est aspirée sans toucher la tête de capteur (3) elle-même, de sorte qu'une position relative prévisible, définie, au moins largement constante, entre la tête de capteur (3) et la feuille (2) se déplaçant aléatoirement sur la tête de capteur (3) vers ou en s'écartant de celle-ci est respectée pendant toute l'opération de mesure.

2. Capteur selon la revendication 1,
**caractérisé en ce que** l'élément de capteur fournit des résultats de mesure indépendants de la distance au moins dans une petite zone d'écart entre la tête de capteur (3) et la feuille (2), la distance entre la tête de capteur (3) et la feuille (2) pouvant se trouver à l'intérieur de la zone d'écart des résultats de mesure indépendants de la distance.

3. Capteur selon la revendication 1 ou 2,
**caractérisé en ce que** l'élément de capteur travaille capacitivement ou inductivement.

4. Capteur selon l'une des revendications 1 à 3,
**caractérisé en ce que** la tête de capteur (3) est montée à coulissement dans la fixation (4).

5. Capteur selon l'une des revendications 1 à 4,
**caractérisé en ce que** l'amenée de gaz (6) est constituée par une amenée d'air comprimé.

6. Capteur selon la revendication 5,
**caractérisé en ce que** le côté mesure de la tête de capteur (3) est réalisé en forme de plaque et que plusieurs ouvertures de sortie (7) sont disposées de préférence concentriquement autour de la position de l'élément de capteur, de préférence au milieu de la plaque, les ouvertures de sortie (7) débouchant de préférence dans des rainures ou des cannelures de la plaque (8) formées du côté mesure.

7. Capteur selon la revendication 5 ou 6,
**caractérisé en ce que** la distance entre la tête de capteur (3) et la feuille (2) est réglable par la pression de gaz en liaison avec le dimensionnement et la disposition de l'ouverture de sortie (7) ainsi que le cas échéant des rainures ou des cannelures.

8. Capteur selon l'une des revendications 5 à 7,
**caractérisé en ce que** la tête de capteur (3) est disposée du côté d'extrémité sur un tube (9) et que l'amenée de gaz (6) est guidée à travers le tube (9), le tube (9) avec la tête de capteur (3) étant coulissant sensiblement sans frottement dans la fixation (4) sur un coussinet d'air et/ou la fixation (4) comprenant un tube de guidage (11) pour le tube (9) et que la paroi du tube de guidage (11) présente des ouvertures de passage (12) pour l'introduction d'air.

9. Capteur selon l'une des revendications 1 à 8,
**caractérisé en ce que** la fixation comprend un dispositif d'avance pour le réglage grossier de la position de la tête de capteur (3), le dispositif d'avance pouvant comprendre un entraînement commandé, un capteur de distance (22) pour le réglage grossier de la position de la tête de capteur (3) pouvant être prévu, qui travaille capacitivement, inductivement, optiquement ou selon le principe de mesure par ultrasons, et le capteur de distance (22) étant disposé à la tête de capteur (3).

10. Capteur selon la revendication 9,
**caractérisé en ce que** l'entraînement du dispositif d'avance se produit pneumatiquement, électriquement ou magnétiquement.

11. Capteur selon la revendication 8 et selon la revendication 10,
**caractérisé en ce qu'**une chambre de pression (14) est formée dans le tube de guidage (11), que le tube (9) est guidé à travers la chambre de pression (14) et est muni dans la zone de la chambre de pression (14), d'une plaque de pression (15) orientée orthogonalement à la direction de déplacement de la tête de capteur (3), que la plaque de pression (15) divise la chambre de pression (14) en deux chambres partielles (16, 17), que chaque chambre partielle (16, 17) présente un raccord à une conduite de gaz (18, 19) et que la conduite de gaz (18, 19) est guidée dans la tête de capteur (3).

12. Capteur selon la revendication 10 ou 11,
**caractérisé en ce qu'**un piézo moteur ou une broche sert d'entraînement.

13. Capteur selon l'une des revendications 1 à 12,
**caractérisé en ce que** des connecteurs sont prévus pour l'alimentation en énergie dans la tête de capteur et/ou la transmission de signaux entre la tête de capteur, ainsi qu'une unité d'exploitation/commande.

14. Capteur selon l'une dès revendication 1 à 12,
**caractérisé en ce que** l'alimentation en énergie dans la tête de capteur se produit sans contact, l'alimentation en énergie se produisant inductivement selon le principe du transformateur.

15. Capteur selon l'une des revendications 1 à 14,
**caractérisé en ce que** la transmission de signaux entre la tête de capteur et une unité d'exploitation/commande se produit sans contact, la transmission de signaux se produisant optiquement, inductivement ou capacitivement, un dispositif émetteur/récepteur (26) pouvant être prévu pour la transmission des signaux et le dispositif émetteur/récepteur (26) comprenant de préférence un dispositif de modulation pour une modulation par impulsions codées, une modulation de fréquence ou une modulation d'amplitude des signaux.

16. Capteur selon la revendication 15,
**caractérisé en ce qu'**un préamplificateur (27) est prévu pour l'amplification du signal de mesure avant la transmission des signaux.
